# EUROPEAN PATENT APPLICATION

(11) **EP 4 403 136 A1**
(43) Date of publication of application: **24.07.2024**
(21) Application number: 23020037.0
(22) Date of filing: 23.01.2023
(51) Int. Cl.: A61F 2/24

(54) **IMPLANT FOR IMPROVING COAPTATION OF AN ATRIOVENTRICULAR VALVE**

(71) Applicant: AVVie GmbH, 1090 Vienna (AT)
(72) Inventor: Mohl, Werner, A-2571 Altenmarkt/Thennenberg (AT); Khienwad, Thananya, A-1190 Vienna (AT)
(74) Representative: Keschmann, Marc

(57) **Abstract**

An implant for improving coaptation of an atrioventricular valve in a human heart, the atrioventricular valve having a first and a second native leaflet, an anterolateral and posteromedial commissure between the first and the second native leaflet, and an annulus adjacent a wall of an atrium of the heart comprising a posterior annulus section and an anterior annulus section, the implant comprising a frame that comprises a lower frame section that frames and supports an artificial leaflet, the artificial leaflet being arranged and configured to cover the first native leaflet, wherein the lower frame section comprises a bent region, in which a bend is formed between a first region of the artificial leaflet and a second region of the artificial leaflet that forms a coaptation plane for coaptation with the second native leaflet, the frame further comprising an upper frame section that frames and supports a flexible structure configured to rest against the wall of the atrium along the posterior annulus section, wherein the upper frame section comprises two end sections located at opposite end regions of the upper frame section, said end sections each protruding from the bend in a circumferential direction of the annulus and configured to rest against the wall of the atrium above the anterior annulus section.

## Description

The invention relates to an implant for improving coaptation of an atrioventricular valve in a human heart.

Atrioventricular valves are membranous folds that prevent backflow from the ventricles of the human heart into the atrium during systole. The valves are anchored within the ventricular cavity by chordae tendineae, which prevent the valves from prolapsing into the atrium when they close. The chordae tendineae are attached to papillary muscles that cause tension to better hold the valve. Together, the papillary muscles and the chordae tendineae constitute the subvalvular apparatus.

The human heart comprises two atrioventricular valves, the mitral valve and the tricuspid valve. The mitral valve allows the blood to flow from the left atrium into the left ventricle. The tricuspid valve is located between the right atrium and the right ventricle.

The mitral valve has an annulus and two leaflets that are each divided into several scallops. The anterior leaflet has three scallops, namely Al, A2 and A3, and the posterior leaflet has three scallops, namely P1, P2 and P3. The anterior and posterior leaflet of the mitral valve are divided by the anterolateral and posteromedial commissure.

The tricuspid valve has three leaflets.

Engagement of the corresponding surfaces of the opposed leaflets against each other, such as, e.g., the engagement of the anterior and posterior leaflet of the mitral valve, is decisive for providing proper valve closure.

Native heart valves become dysfunctional for a variety of pathological causes. Failure of the leaflets to properly close during ventricular systole is known as malcoaptation and causes a backward flow of blood through the valve from the ventricle into the atrium, i.e., in the wrong direction. This physiological process is called regurgitation.

Malcoaptation is often caused by a dilatation of the annulus and the atria and ventricles.

Another cause for malcoaptation may be an excessive motion of the leaflet structures, which is due to local elongation or rupture of the chordae tendineae and resulting in a prolapse of parts of the leaflet into the atrium.

A restriction in motion of the leaflet structures may also result in malcoaptation.

Heart valve regurgitation may result in cardiac failure, decreased blood flow, lower blood pressure, and/or a diminished flow of oxygen to the tissues of the body.

Mitral regurgitation causes an undesired backflow of blood from the left atrium to the pulmonary veins, which in turn may cause congestion and backward heart failure.

One of the main targets in clinical therapy of dysfunctional heart valves is hence to restore proper valve closure.

Proper valve closure may be achieved by complete or partial replacement of the dysfunctional, native heart valve with an artificial heart valve prosthesis.

There are two main types of artificial heart valves, namely mechanical-type and tissue-type valves.

The mechanical-type heart valves make use of mechanical pivoting closure means, which are supported by a base structure and therewith enable coaptation and unidirectional blood flow.

The tissue-type valves have flexible leaflets supported by a base structure and projecting into the flow stream. Tissue-type valves show similar function as native human heart valves and mimic their natural flexing action to coapt against each other.

The instant invention aims at improving state of the art heart implants such that valve closure and valve opening are effectively restored by artificial means, which perfectly mimic native anatomical structures and thereby effectively prevent prolapse of the native leaflet into the atrium.

Further, the present invention aims at providing an improved implant, which effectively withstands the operating forces prevailing in the heart and which is easily to deploy at its target site.

The implant according to the invention also aims at improving state of the art implants such that normal leaflet motion is reproduced, i.e., such that the implant does not restrict leaflet motion of the native leaflet opposed to the implant, and such that the implant does not encourage excessive motion of the opposed leaflet.

Further, the implant shall correct a variety of mitral pathologies, i.e., primary and secondary mitral regurgitation, and mitral prolapse or restrictive mitral function.

The implant according to the invention shall prevent inclination of the implant and migration of the implant into the ventricular cavity.

In addition, the implant according to the invention shall be easy to use, safe, and efficient, enabling a shorter, low-risk surgical intervention and significantly improving patient recovery, thereby reducing the duration of hospitalization.

In order to solve the above objectives, the invention provides an implant for improving coaptation of an atrioventricular valve in a human heart, the atrioventricular valve having a first and a second native leaflet, an anterolateral and posteromedial commissure between the first and the second native leaflet, and an annulus adjacent a wall of an atrium of the heart comprising a posterior annulus section and an anterior annulus section, the implant comprising a frame that comprises a lower frame section that frames and supports an artificial leaflet, the artificial leaflet being arranged and configured to cover the first native leaflet, wherein the lower frame section comprises a bent region, in which a bend is formed between a first region of the artificial leaflet and a second region of the artificial leaflet that forms a coaptation plane for coaptation with the second native leaflet, the frame further comprising an upper frame section that frames and supports a flexible structure configured to rest against the wall of the atrium along the posterior annulus section, wherein the upper frame section comprises two end sections located at opposite end regions of the upper frame section, said end sections each protruding from the bend in a circumferential direction of the annulus and configured to rest against the wall of the atrium above the anterior annulus section.

The invention is based on the idea that dysfunctional valve closure and opening are best restored with an implant, which mimics native leaflet function in an utmost realistic way. This effect may only be achieved by an implant, which provides a satisfactory coaptation surface for the opposing leaflet during systole and which allows for sufficient blood flow during diastole.

To enable an anatomically correct reproduction of a native leaflet, the implant according to the invention is designed such that it covers the dysfunctional native leaflet to a large degree and that it extends into the commissures of the native leaflet, and such that it comprises a bent region, which is, after having been implanted, oriented towards the opposing native leaflet with which coaptation is to be restored.

The implant according to the invention hence perfectly mimics the natural curvature of the diseased native leaflet, e.g., the native shape of scallops P1, P2 and P3 of the mitral posterior leaflet.

The lower frame section comprising the artificial leaflet structure covers the dysfunctional native leaflet.

Preferably, the artificial leaflet forms a coaptation plane with the opposing native leaflet from the anterolateral commissure to the posteromedial commissure with a height of at least 6mm.

The upper frame section of the implant according to the invention provides for an improved stability of the implant when being implanted in the heart.

Because the upper frame section of the implant rests against the wall of the atrium along the posterior annulus section, the implant withstands pressures of varying intensity, which act on the valve during systole and diastole, due to which the implant tends to swing back and forth. The implant according to the invention hence reduces swinging motion, whereby the implant is held stabilized and held in place.

The upper frame section comprises two end sections, which are configured to rest against the wall of the atrium above the anterior annulus section, i.e., the annulus section of the native leaflet opposing the dysfunctional native leaflet, which is covered by the implant according to the invention.

Those end sections prevent an inclination of the implant towards the ventricular cavity and migration of the implant into the ventricular cavity, since the end sections abut against the atrial wall when the implant moves during systole and diastole, whereby the implant is stabilized.

Optimal alignment of the upper frame section with the atrial wall is achieved using the patient's anatomy, segmented from different imaging modalities.

To make the implant even more stable against the forces prevailing in the human heart and to provide for a restricted back-and-forth swinging movement, the end sections of the upper frame section are preferably each curved so as to be curved away from the bend. Said preferred embodiment provides for a uniform rolling motion on the atrial wall since the end sections conform with the anatomy of the atrial wall.

To prevent paravalvular leakage, i.e., an abnormal flow between the valvular annulus and the implant, which is a common complication after valvular replacement surgery due to inadequate sealing, the flexible structure of the upper frame section preferably comprises a first flexible sheet and a second flexible sheet, wherein the first flexible sheet is configured to rest against the wall of the atrium and the second flexible sheet is configured to face away from the wall of the atrium and wherein the first flexible sheet and the second flexible sheet define a tubular cavity capable of being filled with blood. In the tubular cavity the blood which flows towards the annular region may be trapped.

The first flexible sheet preferably has a porous structure to encourage tissue ingrowth on the atrial wall by allowing ingrowth of cells into the porous surface of the flexible sheet.

Alternatively, the first flexible sheet may be provided with a patterning.

The second flexible sheet is preferably made of at least one mesh or a membrane to provide a smooth surface, since the second flexible sheet, i.e., said side of the upper frame, which faces away from the wall of the atrium, needs to be provided with an anticoagulant surface. Thereby thrombus formation may be effectively prevented.

Preferably, the first flexible sheet and the second flexible sheet define a gap between them for allowing blood to enter into the tubular cavity from between the first native leaflet and the artificial leaflet.

To prevent the movement of the implant in the area of the commissures and to thereby provide fixation to the heart tissue, the upper frame section preferably comprises a plurality of first fixing means arranged and configured to engage with the wall of the atrium above the anterolateral and posteromedial commissure.

Preferably, the first fixing means are designed as fixing pins or fixing hooks.

To prevent the migration of the implant in any direction, e.g., towards the ventricle and to hence hold the implant securely in place, the upper frame section preferably comprises a plurality of second fixing means arranged and configured to engage with the wall of the atrium above the posterior annulus section, wherein the second fixing means are preferably arranged in a row extending in a circumferential direction of the posterior annulus section between the artificial leaflet and the flexible structure.

Preferably, the second fixing means are designed as retrievable hooks.

Function and seating of the implant may be monitored after the retrievable hooks are set. If the seating of the implant is not as desired, the hooks may be retrieved and the implant may be repositioned, whereupon the hooks may be again deployed thereby fixing the implant in the new, optimized position.

Preferably, the frame further comprises at least one clamping frame section.

The at least one clamping frame section preferably extends from a distal end of the lower frame section and is arranged and configured to extend on a backside of the first native leaflet and to clamp the first native leaflet or the posterior annulus section between a clamping end of the at least one clamping frame section and the lower frame section.

In addition to the optional fixation by the afore mentioned first and second fixing means, the at least one clamping frame section provides for an additional anchoring point.

The at least one clamping frame section clamps at least the distal end of the dysfunctional native leaflet and hence grabs the free edge of the dysfunctional leaflet.

Alternatively, the at least one clamping frame section may also clamp the dysfunctional leaflet nearly over its entire backside, i.e., the distal end of the at least one clamping frame section is arranged adjacent to the annulus.

To further control potential migration and to prevent rocking of the implant the distal end of the at least one clamping frame section is preferably equipped with a fixing means for fixing the distal end of the clamping frame section to the upper frame section.

The fixing means may be a fixing pin or a transleaflet spike, which pierces the first native leaflet or the posterior annulus section from the ventricular side thereby reaching the atrium, where the upper frame section of the implant is located. After piercing the leaflet or the annulus section, respectively, the fixing pin/transleaflet spike engages with the center of the upper frame section thereby securely holding the implant in place.
A further advantage provided by the at least one clamping frame section is the provided contact of the dysfunctional native leaflet with the ventricular wall, since the impaired native leaflet may be pressed towards the ventricular wall by the clamping frame section, and may hence be hindered to interfere with the movement of the other parts of the implant.

According to the above preferred embodiment, the fixation of the implant is not only dependent on tissue ingrowth of the first flexible sheet of the upper frame section and/or anchoring of the first and second fixing means in the heart tissue, but is enhanced by a ventricular clamp, i.e., the clamping frame section, which ensures alignment of the implant with the dysfunctional native leaflet and fixes the same to the implant.

By the contact of the clamping frame section with the ventricular wall, the implant further serves to prevent an undesired movement of the implant during the cardiac cycle, such as prolapse of the posterior leaflet into the left atrium.

Preferably, the frame is made of a stent structure, preferably a metal stent structure, a shape-memory alloy, such as, e.g., Nitinol, a wire frame, struts, or is a laser cut material, or is in part made by 3D-metal printing.

Preferably, the frame has a super-elastic memory shape.

The artificial leaflet structure is preferably made of a polymer, such as polyurethane, polyamide and ePTFE.

Alternatively, the artificial leaflet structure is preferably made of natural tissue, i.e., pericardial tissue, which is sawn onto the frame.

The artificial leaflet structure may also comprise a permeable, e.g., porous section, and/or an impermeable anticoagulant matrix material.

To provide for a minimal invasive implantation procedure, the frame together with the artificial leaflet and optionally the flexible structure is deployable from a first position, in which the implant is folded for being arranged within a tubular housing of a delivery device, into a second position, in which the implant is deployed.

By the small diameter of the implant in its folded state, the implant may be easily deployed to the heart, e.g., using a transvascular approach.

In particular, the implant may also be advanced into the heart by means of a delivery catheter or a deployment instrument transatrially, transseptally, transfemorally or transapically.

Preferably, the atrioventricular valve is a mitral valve and the first native leaflet is a posterior leaflet of the mitral valve and the second native leaflet is an anterior leaflet of the mitral valve.

Preferably, the artificial leaflet comprises a flexible material, such that the artificial leaflet is able to expand in size during systole and decrease in size during diastole, whereby the distance between the frame of the implant and the highest point of the bulge of the artificial leaflet structure is increased during systole and decreased during diastole.

Due to its expansion properties, the artificial leaflet provides a functioning coaptation surface during systole, which enables proper valve closure and hinders backflow of blood from the ventricle into the atrium.

Contrary thereto, the decreasing properties of the artificial leaflet provide an orifice between the opposing valves, thereby allowing for sufficient blood flow from the atrium to the ventricle during diastole.

The flexible material of the artificial leaflet enables accommodation of the implant to the native leaflet motion, i.e., the native leaflet is not impaired by the artificial leaflet structure, but supported.

A preferred embodiment provides an artificial leaflet structure, which is made of a compliant membrane, which changes its conformation according to the pressure prevailing in the heart, i.e., billows during systole and collapses during diastole.

To mimic the natural shape of, e.g., scallops P1, P2 and P3 of the posterior leaflet of the mitral valve, the artificial leaflet is preferably built as flexible polymer cup-like coverings.

To enable full coverage of the dysfunctional native leaflet of a mitral or tricuspid valve, the artificial leaflet may preferably comprise a first lateral segment, a middle segment and a second lateral segment.

By said preferred embodiment full coverage of the dysfunctional native leaflet by a three-part, i.e., segmented artificial leaflet structure is enabled.

The first lateral segment of the artificial leaflet may cover scallop P1 of the native, dysfunctional leaflet. The middle segment of the artificial leaflet may cover scallop P2 of the native, dysfunctional leaflet. The second lateral segment of the artificial leaflet may cover scallop P3 of the native, dysfunctional leaflet.

The three segments are preferably formed as a one-piece leaflet structure or, alternatively may be formed by three individual interconnected leaflet structures.

To better mimic the anatomical structure of scallops P1, P2 and P3 during systole and diastole, the individual segments of the artificial leaflet are preferably bulgeable, whereby the middle segment of the artificial leaflet may bulge to a larger extent than the lateral segments of the artificial leaflet.

According to said preferred embodiment, the individual segments of the artificial leaflet are hence formed as pillow-like structures, which bulge, whereby their bulges increase in size due to the blood pressure during systole resulting in valve closure, and decrease in size due to the blood pressure during diastole resulting in the opening of the valve.

The part of the implant, which overlays the distal end of the dysfunctional leaflet opposite the annulus hence may range in size from small surfaces to large bulges in the membrane, thereby mimicking, e.g., P1, P2 and P3 of the posterior leaflet of the mitral valve.

In addition, or alternatively to the compliant membrane of the artificial leaflet, the frame of the implant may be compliant.

Compliance of the frame may, e.g., be achieved by thermoforming techniques. For being compliant, the frame may be designed such that one part of the frame, e.g., the upper frame section, exerts a slight outward radial force on the annulus, which ensures maintenance of the contact between the implant and the annulus.

Preferably, the frame is selectively compliant, thereby providing sufficient support for the artificial leaflet of the implant by being stiff in some of the regions of the frame, whilst still accommodating to changes in valve geometry throughout the cardiac cycle due to flexible regions of the frame.

Selective compliance in the sense of the present invention is to be understood such that the implant shows a higher stability in regions where large support is needed, and is more flexible in regions, which must adapt to the anatomical conditions acting upon the valve.

The middle segment of the artificial leaflet structure preferably differs in size from the first and second lateral segments of the artificial leaflet, which provides for an optimized 3D-shape of the implant, and therefore provides for perfect imitation of the anatomical structure, i.e., mimics scallops P1, P2 and P3 of an unimpaired native mitral valve leaflet, in which scallop P2 is larger in size than P1 and P3.

Alternatively, when the implant is to be used to repair an anterior leaflet prolapse, the artificial central leaflet comprises a single component divided in scallop A1, A2 and A3 mimicking structures.

The invention will now be described in more detail with reference to the attached drawings.

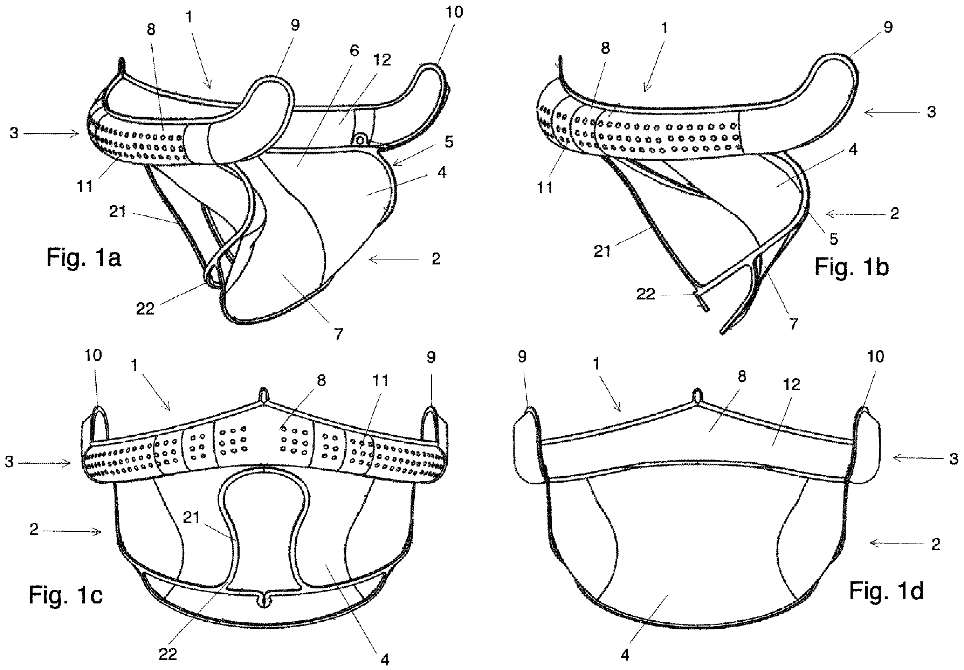

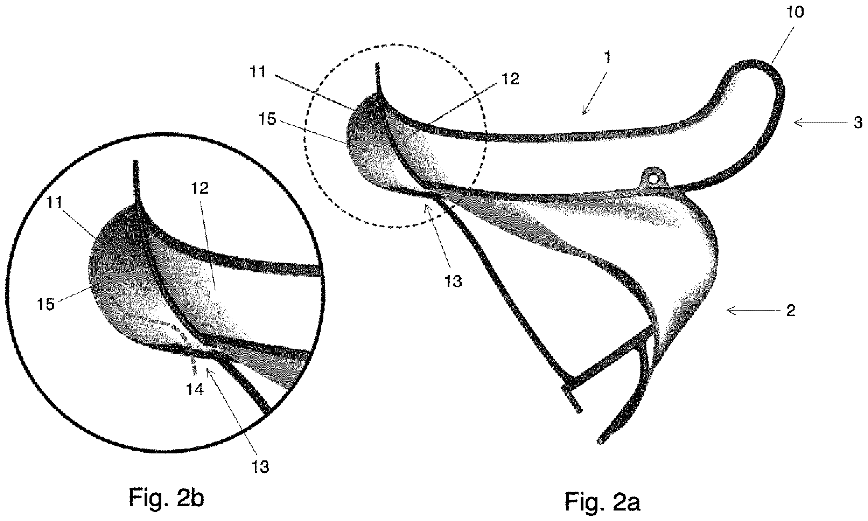

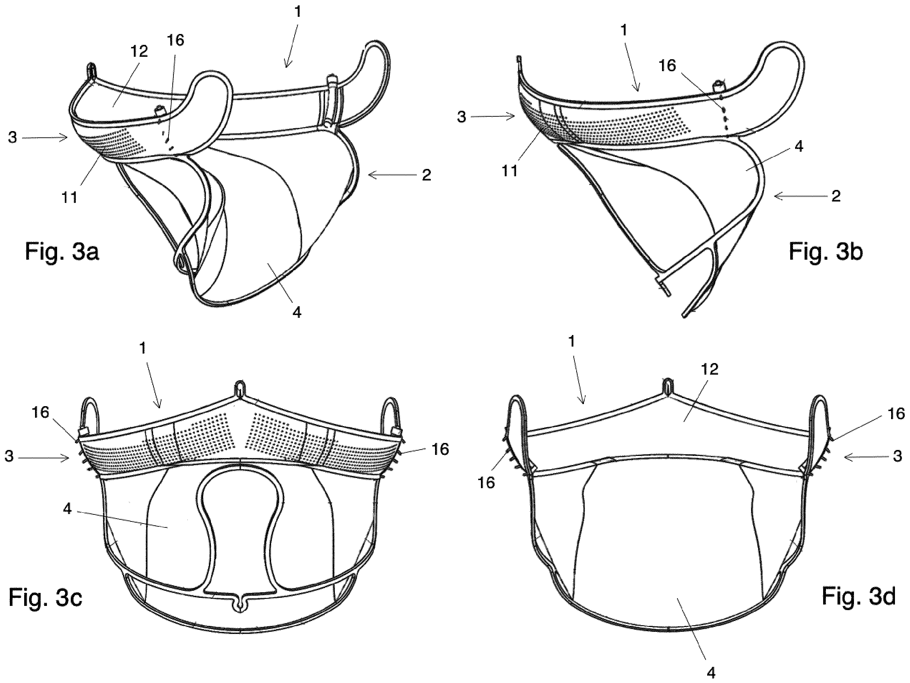

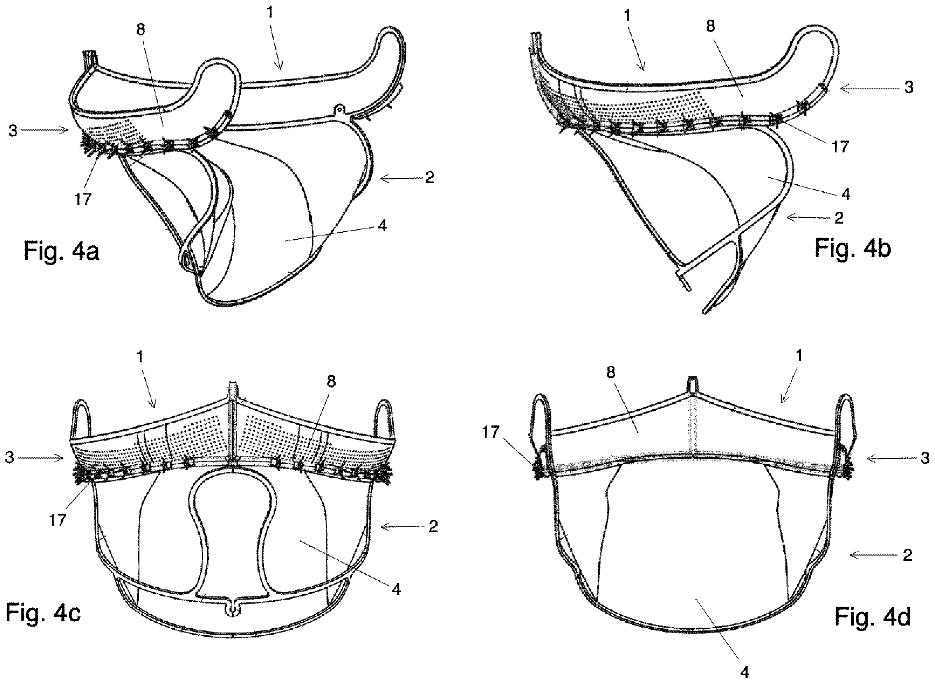

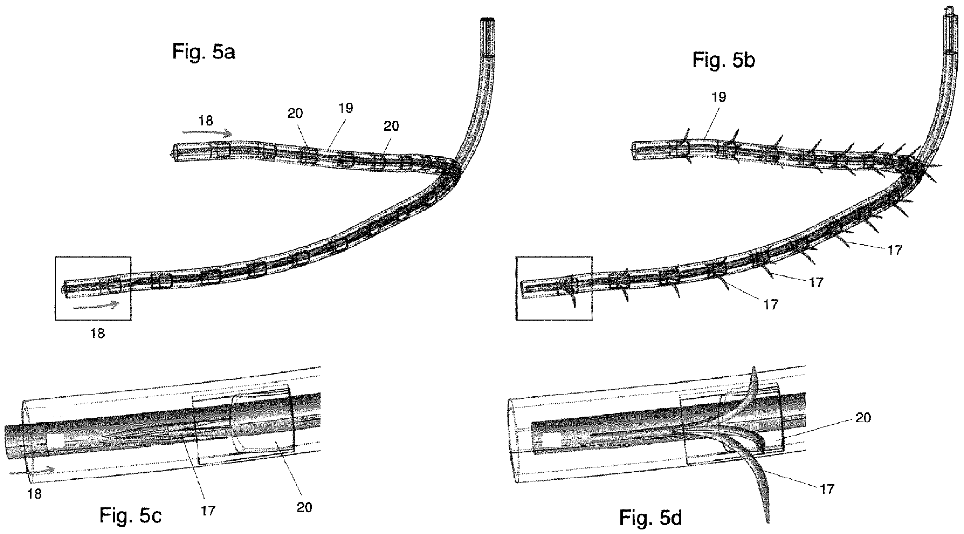

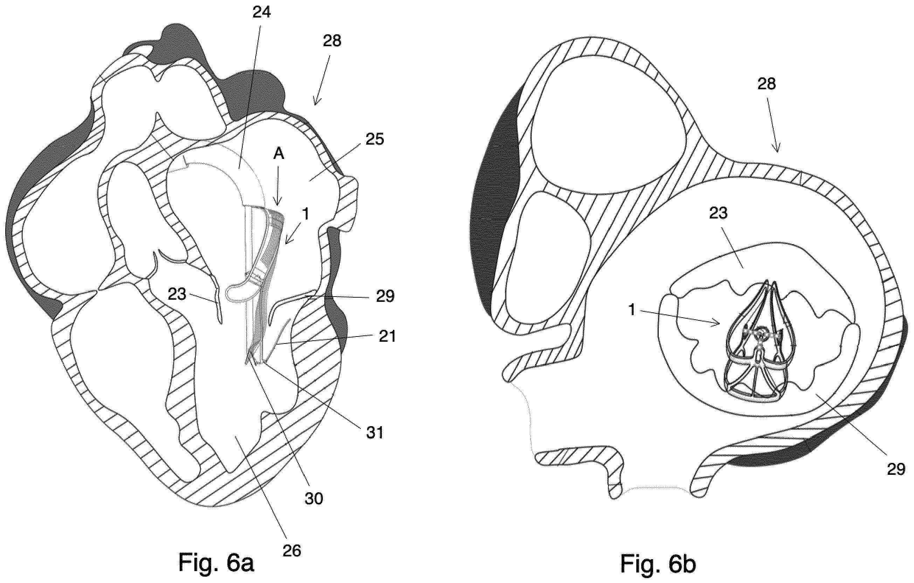

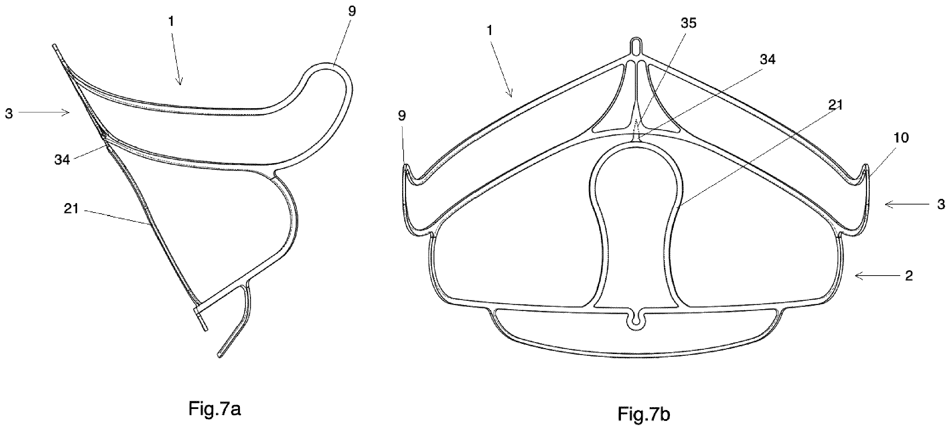

In the drawings, Fig.1a-d shows a first embodiment of the implant according to the invention, Fig.2a and b show a detailed view of the first flexible sheet and the second flexible sheet of the upper frame section of the implant according to the first embodiment as depicted in Fig.1, Fig.3a-d show a second embodiment of the implant according to the invention, Fig.4a-d show a third embodiment of the implant according to the invention, Fig.5a-d show a detailed view of the second fixing means of the third embodiment when being designed as retrievable hooks, and Fig.6a-f show the implant according to the first embodiment of the invention when being implanted to the mitral valve of a human heart. Fig.7a-b show a fourth embodiment of the implant according to the invention.

Fig.1a-d show different views of a first embodiment of the implant according to the invention, in which the implant is denoted by arrow 1. Fig.1a shows a perspective view, Fig.1b shows a side view, Fig.1c shows a rear view and Fig.1d shows a front view of the implant 1. The implant 1 comprises a lower frame section 2 and an upper frame section 3.

The lower frame section 2 frames and supports an artificial leaflet 4, and comprises a bent region, in which a bend 5 is formed between a first region 6 of the artificial leaflet 4 and a second region 7 of the artificial leaflet 4, whereby a coaptation plane for coaptation with the second native leaflet is formed.

The upper frame section 3 frames and supports a flexible structure 8 configured to rest against the wall of the atrium along the posterior annulus section, and comprises two end sections 9,10, which are located at opposite end regions of the upper frame section 3, and are configured to rest against the wall of the atrium above the anterior annulus section. As can be taken from Fig.1, the end sections 9,10 of the upper frame section 3 are protruding from the bend 5 in a circumferential direction of the annulus and are curved away from the bend 5.

The flexible structure 8 of the upper frame section 3 comprises a first flexible sheet 11 with a porous structure and a second flexible sheet 12 with a smooth structure.

The implant 1 further comprises a clamping frame section 21, which extends from a distal end 22 of the lower frame section 2 and is arranged and configured to extend on a backside of the first native leaflet and to clamp the first native leaflet or the posterior annulus section between a clamping end of the clamping frame section 21 and the lower frame section 2.

Fig.2a shows a side view of the implant 1 of Fig.1 and Fig.2b shows a detailed view of the first flexible sheet 11 and the second flexible sheet 12 of the upper frame section 3 of the implant 1 according to the first embodiment as depicted in Fig.1.

The first flexible sheet 11 and the second flexible sheet 12 define a gap 13 between them for allowing blood 14 to enter into a tubular cavity 15.

Fig.3a-d show a second embodiment of the implant 1 according to the invention, which is equipped with a plurality of first fixing means 16 on its upper frame section 3, which are arranged and configured to engage with the wall of the atrium above the anterolateral and posteromedial commissure. Thereby the seating of the implant on the level of the annulus is secured. As can be taken from Fig.3 the first fixing means 16 are designed as fixing pins, which penetrate the heart tissue. Herein, Fig.3a shows a perspective view, Fig.3b shows a side view, Fig.3c shows a rear view and Fig.3d shows a front view of the implant 1.

Fig.4a-d show a third embodiment of the implant 1 according to the invention. Fig.4a shows a perspective view, Fig.4b shows a side view, Fig.4c shows a rear view and Fig.4d shows a front view of the implant 1. In the third embodiment, the upper frame section 3 comprises a plurality of second fixing means 17, which are arranged in a row extending in a circumferential direction of the posterior annulus section of the implant 1, whereby the second fixing means 17 are arranged between the artificial leaflet 4 of the lower frame section 2 and the flexible structure 8 of the upper frame section 3 extending into the atrial region above the anterior leaflet.

Fig.5a-d show a detailed view of the second fixing means 17 as depicted in Fig.4. Herein, Fig.5a shows a retracted position of the fixing means 17, Fig.5b shows a deployed position of the fixing means 17, Fig.5c shows a detail of Fig.5a and Fig.5d shows a detail of Fig.5b. As can be taken from Fig.5 the second fixing means 17 are designed as retrievable hooks with a trident shape. The retrievable hooks are pushed out of an annulus tube 19 with a pusher in the direction of arrow 18 and deploy when exiting windows 20 of the annulus tube 19. This feature allows to retrieve the hooks in case of malpositioning.

Fig.6a-f show the implant 1 according to the first embodiment of the invention when being implanted in the mitral valve of a human heart 28. The posterior leaflet of the mitral valve is denoted with 29 and the anterior leaflet is denoted with 23.

Fig.6a shows that the implant 1 is advanced through a steerable catheter 24 into the left atrium 25 and is moved towards the left ventricle 26 until the orifice between the anterior leaflet 23 and the posterior leaflet 29 is reached.

The clamping frame section 21 of the implant 1 is then gently released from the catheter 24 and precisely aligned at the center of the posterior prolapsed leaflet 29 from the ventricular side.

The alignment takes place via activation of the clasp rocker 30, which is anchored via a connector hole 31 to the clamping frame section 21.

After aligning the clamping frame section 21 the catheter 24 is slowly slid up above the mitral leaflet plane, resulting in positioning the clamping frame section 21 between the prolapsed posterior leaflet 29 and the inner wall of the left ventricle 26.

Fig.6b shows a top view of the implantation status of Fig.6a according to arrow A.

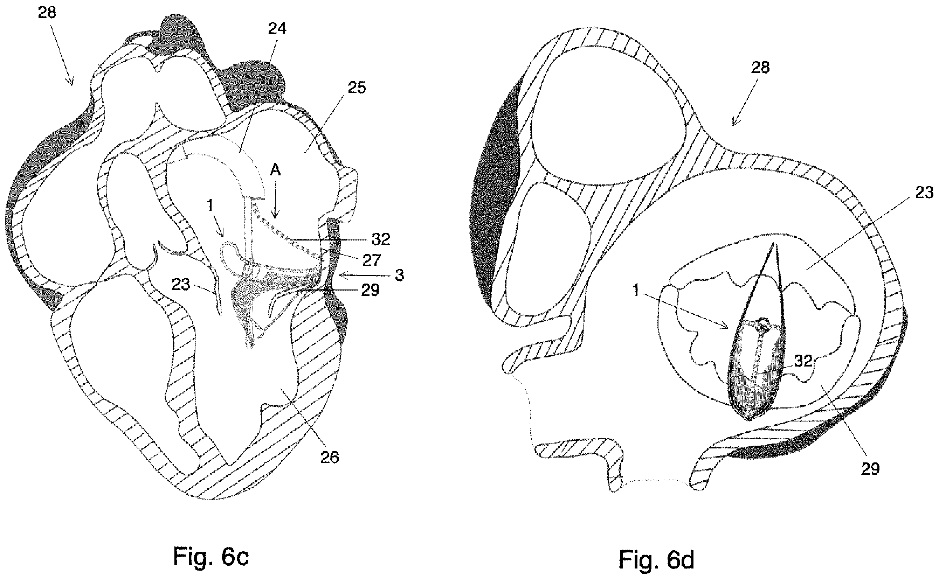

Once the position of the clamping frame section 21 is secured, the upper frame section 3 of the implant 1 is actively swung towards the left atrial wall 27 by the aid of the atrial rocker 32, whereby the upper frame section 3 is positioned at the annular portion of the center of the posterior leaflet 29 (Fig.6c).

Fig.6d shows a top view of the implantation status of Fig.6c according to arrow A.

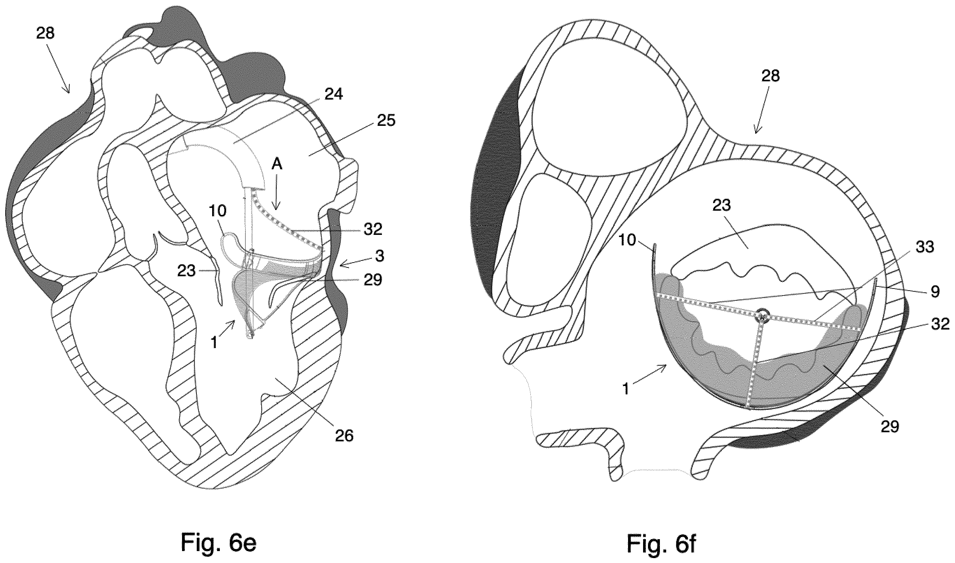

Fig.6e and Fig.6f show the final step of the implantation of the implant 1, in which the commissural pushers 33 are used to fully deploy the implant 1 and to precisely push the two end sections 9,10 of the upper frame section 3 into the commissures, i.e., the area in which the anterior leaflet 23 and the posterior leaflet 29 abut.

After placing the implant 1 in its desired position, the fixing means, which are described and depicted in Fig.3a-d and Fig.4a-d, are deployed to fix the implant 1 to the heart tissue and to prevent migration of the implant 1.

Finally, the implant 1 is released from the steerable catheter 24 and the catheter is withdrawn.

Fig.6f shows a top view of the implantation status of Fig.6e according to arrow A.

Fig.7a-b show a fourth embodiment of the implant 1 according to the invention. Fig.7a shows a side view, Fig.7b shows a rear view.

In the fourth embodiment, the clamping frame section 21 of the implant 1 is equipped with a fixing means 34 on its distal end. The fixing means 34 engages with a recess 35 in the center of the upper frame section 3 thereby connecting the clamping frame section 21 with the upper frame section 3.

When being fixed to the first native leaflet the fixing means 34 punctures the leaflet, which is arranged between the upper frame section 3 and the clamping frame section 21, and then engages with the recess 35. Thereby the implant 1 is fixed to the native leaflet and securely hold in place.

## Claims

1. An implant for improving coaptation of an atrioventricular valve in a human heart, the atrioventricular valve having a first and a second native leaflet, an anterolateral and posteromedial commissure between the first and the second native leaflet, and an annulus adjacent a wall of an atrium of the heart comprising a posterior annulus section and an anterior annulus section, the implant comprising a frame that comprises a lower frame section that frames and supports an artificial leaflet, the artificial leaflet being arranged and configured to cover the first native leaflet, wherein the lower frame section comprises a bent region, in which a bend is formed between a first region of the artificial leaflet and a second region of the artificial leaflet that forms a coaptation plane for coaptation with the second native leaflet, the frame further comprising an upper frame section that frames and supports a flexible structure configured to rest against the wall of the atrium along the posterior annulus section, wherein the upper frame section comprises two end sections located at opposite end regions of the upper frame section, said end sections each protruding from the bend in a circumferential direction of the annulus and configured to rest against the wall of the atrium above the anterior annulus section.

2. Implant according to claim 1, wherein the end sections of the upper frame section are each curved so as to be curved away from the bend.

3. Implant according to claim 1 or 2, wherein the flexible structure of the upper frame section comprises a first flexible sheet and a second flexible sheet, wherein the first flexible sheet is configured to rest against the wall of the atrium and the second flexible sheet is configured to face away from the wall of the atrium and wherein the first flexible sheet and the second flexible sheet define a tubular cavity capable of being filled with blood.

4. Implant according to claim 3, wherein the first flexible sheet has a porous structure.

5. Implant according to claim 3 or 4, wherein the second flexible sheet is made of at least one mesh or a membrane.

6. Implant according to claim 3, 4 or 5, wherein the first flexible sheet and the second flexible sheet define a gap between them for allowing blood to enter into the tubular cavity from between the first native leaflet and the artificial leaflet.

7. Implant according to any one of claims 1 to 6, wherein the upper frame section comprises a plurality of first fixing means arranged and configured to engage with the wall of the atrium above the anterolateral and posteromedial commissure.

8. Implant according to claim 7, wherein the first fixing means are designed as fixing pins or fixing hooks.

9. Implant according to any one of claims 1 to 8, wherein the upper frame section comprises a plurality of second fixing means arranged and configured to engage with the wall of the atrium above the posterior annulus section, wherein the second fixing means are preferably arranged in a row extending in a circumferential direction of the posterior annulus section between the artificial leaflet and the flexible structure.

10. Implant according to claim 9, wherein the second fixing means are designed as retrievable hooks.

11. Implant according to any one of claims 1 to 10, wherein the frame further comprises at least one clamping frame section.

12. Implant according to claim 11, wherein the at least one clamping frame section extends from a distal end of the lower frame section and is arranged and configured to extend on a backside of the first native leaflet and to clamp the first native leaflet or the posterior annulus section between a clamping end of the at least one clamping frame section and the lower frame section.

13. Implant according to claim 12, wherein a distal end of the at least one clamping frame section is equipped with a fixing means for fixing the distal end of the clamping frame section to the upper frame section.

14. Implant according to any one of claims 1 to 13, wherein the frame is made of a stent structure, preferably a metal stent structure, a shape-memory alloy, such as, e.g., Nitinol, a wire frame, struts, or is a laser cut material, or is in part made by 3D-metal printing.

15. Implant according to any one of claims 1 to 14, wherein the frame together with the artificial leaflet and optionally the flexible structure is deployable from a first position, in which the implant is folded for being arranged within a tubular housing of a delivery device, into a second position, in which the implant is deployed.

16. Implant according to any one of claims 1 to 15, wherein the atrioventricular valve is a mitral valve and the first native leaflet is a posterior leaflet of the mitral valve and the second native leaflet is an anterior leaflet of the mitral valve.

17. Implant according to any one of claims 1 to 16, wherein the artificial leaflet comprises a flexible material, such that the artificial leaflet is able to expand in size during systole and decrease in size during diastole.

18. Implant according to any one of claims 1 to 17, wherein the artificial leaflet comprises a first lateral segment, a middle segment and a second lateral segment.
